# EUROPEAN PATENT SPECIFICATION

(11) **EP 2 011 492 B1**
(45) Date of publication and mention of the grant of the patent: **03.10.2012**
(21) Application number: 07737021.1
(22) Date of filing: 04.04.2007
(51) Int. Cl.: A61K 31/215, A61P 1/14, A61P 43/00, A23L 1/30, A23L 2/00

(54) **GIP SECRETION INHIBITOR**
GIP-SEKRETIONSHEMMER
INHIBITEURS DE LA SÉCRÉTION DU GIP

(30) Priority: 24.04.2006 JP 2006119048
(43) Date of publication of application: 07.01.2009
(73) Proprietor: KAO CORPORATION, Chuo-ku Tokyo 103-8210 (JP)
(72) Inventor: TAKENO, Nanami, Ina-shi, Nagano 3960021 (JP); SHIMOTOYODOME, Akira, Haga-gun, Tochigi 321-3497 (JP); MEGURO, Shinichi, Sumida-ku, Tokyo 1318501 (JP)
(74) Representative: Vossius & Partner
(86) International application number: PCT/JP2007/000364
(87) International publication number: WO 2007/122801

(56) References cited:
- JP-A- 05 310 567
- JP-A- 08 193 028
- JP-A- 2001 064 672
- JP-A- 2001 354 558
- JP-A- 2002 138 296
- JP-A- 2002 138 297
- JP-A- 2004 359 784
- DATABASE WPI Week 200707 Thomson Scientific, London, GB; AN 2007-065151 XP002534880 & JP 2006 342084 A (KAO CORP) 21 December 2006 (2006-12-21)
- DATABASE WPI Week 200709 Thomson Scientific, London, GB; AN 2007-086835 XP002535396 & JP 2006 342085 A (KAO CORP) 21 December 2006 (2006-12-21)
- OSILESI O ET AL: "USE OF XANTHAN GUM IN DIETARY MANAGEMENT OF DIABETES MELLITUS" AMERICAN JOURNAL OF CLINICAL NUTRITION, vol. 42, no. 4, 1985, pages 597-603, XP002535395 ISSN: 0002-9165
- ZHOU H. ET AL.: 'Gastric inhibitory polypeptide modulates adiposity and fat oxidation under diminished insulin action' BIOCHEM. BIOPHYS. RES. COMMUN. vol. 335, no. 3, 2005, pages 937 - 942, XP005100410
- SARSON D L: "Gastric inhibitory polypeptide (GIP).", JOURNAL OF CLINICAL PATHOLOGY. SUPPLEMENT (ASSOCIATION OF CLINICAL PATHOLOGISTS) 1978 LNKD- PUBMED:399609, vol. 8, 1978, pages 31-37, ISSN: 0144-0349

## Description

### Technical Field

The present invention relates to an agent for inhibiting GIP secretion which is a useful drug or food ingredient.

### Background Art

According to national nutrition survey in Japan, in modern dietary habits, total caloric intake has remained almost the same since 1960, but lipid intake has increased considerably from 11% of total caloric intake to 27%. Conceivably, such a high-fat diet imposes a burden on the stomach, and causes, for example, a heavy feeling in the stomach (hereinafter referred to as "heavy stomach").

Gastric inhibitory polypeptide (GIP) is a gastrointestinal hormone which is known to exhibit an inhibitory effect on secretion of gastric acid or on gastric motility. As has been known, during consumption of a diet, secretion of GIP is enhanced by, for example, lipid contained in the diet (Non-Patent Documents 1 to 3). Therefore, inhibition of secretion of GIP is considered effective for promoting digestion or alleviating heavy stomach. Previous studies have shown that 3-bromo-5-methyl-2-phenylpyrazolo[1,5-a]pyrimidine-7-ol (BMPP) is a substance which inhibits the function of GIP, and that, for example, guar gum is a substance which inhibits secretion of GIP (Patent Document 1 and Non-Patent Documents 4 to 9). However, BMPP has not been shown to exhibit an inhibitory effect on GIP function in vivo, whereas guar gum has not been studied for its inhibitory effect on GIP secretion upon consumption of lipid, and is not satisfactorily effective in alleviating heavy stomach feeling or other functions. Further, such a GIP secretion inhibitor is demanded to have high safety to the extent that it can be consumed daily.

Monoacylglycerol (hereinafter may be abbreviated as "MAG") is a substance which exhibits high safety and is widely used as, for example, an emulsifier in the field of food. Generally, monoacylglycerol is incorporated into, for example, margarine, milk beverage, ice cream, or bread in an amount of about 0.2 to about 0.5% (Non-Patent Documents 10 and 11). As has been known, monoacylglycerol has an effect in suppressing a postprandial increase in blood triglyceride level (Patent Document 2).
However, the relationship between monoacylglycerol and GIP secretion has not yet been determined.
Patent Document 1: WO 01/87341 pamphlet
Patent Document 2: JP-A-1993-310567
Non-Patent Document 1: J. C. Brown, *et al*., Canadian J. Physiol. Pharmacol. 47: 113-114, 1969
Non-Patent Document 2: J. M. Falko, *et al*., *J*. Clin. Endocrinol. Metab. 41 (2): 260-265, 1975
Non-Patent Document 3: Toshitsugu Oda, et al., Shokakan, Kino to Byotai ("Gastrointestinal Tract, Function and Pathological Condition"), 1981, Chugai-Igakusha, pp. 205-216
Non-Patent Document 4: Gagenby S J, et al., Diabet. Med. 1996 Apr.; 13 (4): 358-64
Non-Patent Document 5: Ellis PR, et al., Br. J. Nutr. 1995 Oct.; 74 (4): 539-56
Non-Patent Document 6: Simoes Nunes C, et al., Reprod. Nutr. Dev. 1992; 32 (1) : 11-20
Non-Patent Document 7: Morgan LM, et al., Br. J. Nutr. 1990 Jul.; 64 (1): 103-10
Non-Patent Document 8: Requejo F, et al., Diabet. Med. 1990 Jul.; 7 (6): 515-20
Non-Patent Document 9: Morgan, et al., Br. J. Nutr. 1985 May; 53 (3): 467-75
Non-Patent Document 10: Kazuhiro Okamura, et al., Shokuhin Tenkabutsu no Siyoho ("Method for Use of Food Additive"), 1973, Shokuhin to Kagakusha, pp. 288-289
Non-Patent Document 11: Seiji Fujii, et al., Syokuhin Tenkabutsu Handobukku ("Food Additive Handbook"), 1997, Koseikan Co., Ltd., pp. 236-238

### Summary of the Invention

The present invention provides the following inventions 1) to 7).
1. An agent for use in inhibiting postprandial GIP secretion, which agent comprises a monoacylgylcerol having a glycerine of which the hydroxyl group of 1-position, 2-position or 3-position is esterified with an unsaturated fatty acid having 16 to 22 carbon atoms.
2. An agent for use in promoting digestion, which agent comprises a monoacylgylcerol having a glycerine of which the hydroxyl group of 1-position, 2-position or 3-position is esterified with an unsaturated fatty acid having 16 to 22 carbon atoms.
3. An agent for use in alleviating heavy stomach, which agent comprises a monoacylgylcerol having a glycerine of which the hydroxyl group of 1-position, 2-position or 3-position is esterified with an unsaturated fatty acid having 16 to 22 carbon atoms.
4. The agent for use according to points 1 to 3, which agent comprises a monoacylgylcerol having a glycerine of which the hydroxyl group of 1-position is esterified with an unsaturated fatty acid having 16 to 22 carbon atoms.
5. The agent for use according to points 1 to 4 which is a drug.
6. The agent for use according to points 1 to 4 which is a food ingredient.
7. The agent for use according to points 1 to 4 which is a beverage ingredient.

### Detailed Description of the Invention

The present invention is directed to an agent for inhibiting GIP secretion which is a useful drug or food ingredient.

The present inventors have found that a monoacylglycerol considerably inhibits postprandial GIP secretion, and is effective for promoting digestion or alleviating heavy stomach.

The agent for inhibiting GIP secretion of the present invention can reduce postprandial GIP level, can promote digestion and absorption of diet, and can ameliorate stomach conditions (e.g., can alleviate heavy stomach).

Examples of the monoacylglycerol employed in the present invention include a monoacylglycerol having a glycerin of which the hydroxyl group of the 1 - position is esterified (1-monoacylglycerol) ; with a fatty acid a monoacylglycerol having a glycerin of which the hydroxyl group of the 2 - position is esterified with a fatty acid (2-monoacylglycerol); and a monoacylglycerol having a glycerin of which the hydroxyl group of the 3- position is esterified (3-monoacylglycerol). Preferably, with a fatty acid a 1-monoacylglycerol is employed. No particular limitation is imposed on the number of carbon atoms of the fatty acid residue, but the number is preferably 8 to 24, more preferably 16 to 22. Examples of the fatty acid residue include saturated fatty acid residues and unsaturated fatty acid residues. Specific examples include acyl groups derived from caprylic acid, capric acid, lauric acid, myristic acid, palmitic acid, stearic acid, oleic acid, linoleic acid, linolenic acid, eicosapentaenoic acid, and docosahexaenoic acid; acyl groups derived from a mixture of the aforementioned fatty acids; and acyl groups derived from fatty acids from oils containing the aforementioned fatty acids (e.g., animal oils such as beef tallow and lard, and vegetable oils such as palm oil, rapeseed oil, soybean oil, safflower oil, corn oil, shiso oil, stillingia oil, linseed oil, and perilla oil) . The monoacylglycerols may be employed singly or in combination of two or more species.

When these monoacylglycerols are employed in combination, the ratio of the amount of the unsaturated fatty acid residues to the total amount of the entire fatty acid residues in the monoacylglycerol is preferably 55% or more, more preferably 70% or more, still more preferably 90% or more. Preferably, the unsaturated fatty acid residues include oleic acid residues in an amount of 15 to 85%, and linoleic acid residues in an amount of 15 to 85%. More preferably, the unsaturated fatty acid residues include oleic acid residues in an amount of 50 to 100%. The amount of fatty acid residues constituting the monoacylglycerol is estimated by converting the amount of acyl group constituting the monoacylglycerol to the amount of fatty acids.

The monoacylglycerol employed in the present invention may be produced through any reaction; for example, hydrolysis of an oil containing an unsaturated acyl group, such as linseed oil, perilla oil, shiso oil, soybean oil, or rapeseed oil; transesterification of glycerin with the oil as described above; or esterification of glycerin with a fatty acid derived from the oil as described above. The reaction may be carried out through a chemical technique employing an alkali catalyst or a similar catalyst, or through a biochemical technique employing an enzyme such as lipase. The resultant reaction product may be subjected to fractionation for isolation of a desired monoacylglycerol.

As described in the Example hereinbelow, when monoacylglycerol is consumed together with triacylglycerol (hereinafter may be abbreviated as "TAG"), monoacylglycerol exhibits the effect in significantly suppressing an increase in secretion of GIP caused by consumption of glucose or triacylglycerol. Therefore, monoacylglycerol can be used as an agent for inhibiting postprandial GIP secretion in a food or drug for human or animals, or as an ingredient for such a food or drug.
As used herein, "inhibition of postprandial GIP secretion" refers to suppression of an increase in GIP secreted from the gastrointestinal tract caused by consumption of a diet containing lipid and carbohydrate, preferably, a diet containing a large amount of lipid, more preferably, a diet containing a large amount of triacylglycerol.

Rewarding the agent for inhibiting postprandial GIP secretion of the present invention, the monoacylglycerol of the present invention may be administered singly to a human or an animal, or the monoacylglycerol may be incorporated into, for example, a food, a beverage, a drug, or a pet food which a subject consumes. The agent for inhibiting postprandial GIP secretion may be employed in the form of a food or beverage with a label indicating that it contains monoacylglycerol, exhibits the effect in promoting digestion or alleviating heavy stomach, and is consumed for promoting digestion or alleviating heavy stomach. The agent for inhibiting postprandial GIP secretion may be applied to a food or beverage such as food for beauty purposes, food for the sick, or food for specified health use labeled that it is consumed for amelioration of stomach conditions, such as promotion of gastric acid secretion, promotion of digestion or alleviation of heavy stomach. When the agent for inhibiting postprandial GIP secretion is employed in the form of a drug, the agent may be prepared into a solid dosage form for oral administration such as tablets or granules or a liquid dosage form for oral administration such as a liquid for internal use or a syrup.

A solid dosage form for oral administration such as tablets, coated tablets, granules, powder, or capsules can be prepared by adding, to the monoacylglycerol of the present invention, an excipient and, if necessary, an additive such as a binder, a disintegrating agent, a lubricant, a coloring agent, a sweetening agent, or a flavoring agent, followed by customary processing. A liquid dosage form for oral administration such as liquid for internal use, a syrup, or an elixir can be prepared by adding, to the monoacylglycerol of the present invention, an additive such as a sweetening agent, a buffer, a stabilizer, or a sweetening agent, followed by customary processing.
The amount of the monoacylglycerol contained in any of the aforementioned dosage forms is generally 0.1 mass% or more, preferably 1 mass% or more, more preferably 5 wt.% or more, on the basis of the entire mass of the composition.

The effective dosage (or amount consumed) of the aforementioned dosage forms is preferably 0.01 to 10 g per day as reduced to monoacylglycerol. The agent for inhibiting postprandial GIP secretion of the present invention is effectively dosed or consumed before, during, or after a meal.

### Examples

### Example 1 Inhibitory effect of monoacylglycerol on GIP secretion

The following experiment was carried out by using triolein as a triacylglycerol (TAG), and 1-monoolein as a monoacylglycerol (MAG).
Male C57BL/6J mice (eight weeks old) were divided into groups (12 or 14 mice each). Through a probe, mice of the respective groups were orally administered the following substance: only glucose (2 mg/g-body weight) (Glucose); glucose (2 mg/g-body weighs) plus triolein (2 mg/g-body weight) emulsified with egg yolk lecithin (0.02 mg/g-body weight) (TAG1); TAG1 supplemented with 1-monoolein of 0.08, 0.2 and 0.4 mg/g-body weight, respectively (MAG1, MAG2 and MAG3). Table 1 shows the compositions of the emulsions. Ten minutes after the oral administration, blood was collected from each mouse via the abdominal vena cava, and the blood GIP level was measured through ELISA (Gastric Inhibitory Peptide EIA Kit, Phoenix Pharmaceutical Inc.). Table 2 shows the increment of blood GIP level in all the mice measured 10 minutes after the oral administration, in which the blood GIP level of mice not administered the emulsion is assumed as an initial level.

**[Table 1]**

| Composition of emulsion | Glucose (mg/g-body weight) | Triolein (mg/g-body weiqht) | 1-Monoolein (mg/g-body weight) |
|---|---|---|---|
| Glucose | 2.0 | 0.0 | 0.0 |
| TAG1 | 2.0 | 2.0 | 0.0 |
| MAG1 | 2.0 | 2.0 | 0.08 |
| MAG2 | 2.0 | 2.0 | 0.2 |
| MAG3 | 2.0 | 2.0 | 0.4 |

**[Table 2]**

| Composition of emulsion | Mouse blood GIP level (ng/mL) |
|---|---|
| Glucose | 0.6 ± 0.1 (N = 12) |
| TAG 1 | 1.5 ± 0.2*** (N = 12) |
| MAG1 | 1.3 ± 0.2** (N = 14) |
| MAG2 | 1.2 ± 0.1* (N = 14) |
| MAG3 | 1.0 ± 0.2# (N = 14) |

| | |
|---|---|
| Statistically significant difference in mouse blood GIP level between the Glucose group and the TAG1, MAG1 or MAG2 group: ***: P <0.001, **: P < 0.01, *: P < 0.05 Statistically significant difference in mouse blood GIP level between the TAG1 group and the MAG3 group: #: P < 0.05 Statistically significant difference in concentration dependence of the effect of MAG: P < 0.05 | |

As shown in Table 2, administration of TAG to mice increased blood GIP level as compared to administration of glucose only. However, when MAG were added to TAG1 (MAG1, MAG2, or MAG3) and administered to mice, the increase in blood GIP level caused by administration of TAG was statistically significantly suppressed, and the suppressive effect increased with the concentration of MAG. These data indicate that MAG exhibits an inhibitory effect on GIP secretion. Specifically, for example, in the mice received MAG in an amount of 1/5 of that of TAG, an increase in blood GIP level is suppressed as compared to the case of the mice not received MAG, indicating an inhibitory effect of MAG on GIP secretion in blood.

**[Table 3]**

| Formulation Example (1) Coffee beverage | |
|---|---|
| 1-MAG | 0.1 mass% |
| Coffee bean | 5.5 mass% |
| Milk | 7.0 mass% |
| Sugar | 6.0 mass% |
| Flavor | Slight amount |
| Sodium bicarbonate | (Adjusted to pH 6.5) |
| Water | Balance |

**[Table 4]**

| Formulation Example (2) Candy | |
|---|---|
| 1-MAG | 0.1 mass% |
| Sucrose ester (emulsifier) | 0.2 mass% |
| Thick malt syrup | 35 mass% |
| Sugar | 35 mass% |
| Flour | 5 mass% |
| Condensed milk | 17 mass% |
| Milk | 6 mass% |
| Butter | 2 mass% |
| Flavor | Appropriate amount |

## Claims

1. An agent for use in inhibiting postprandial GIP secretion, which agent comprises a monoacylgylcerol having a glycerine of which the hydroxyl group of 1-position, 2-position or 3-position is esterified with an unsaturated fatty acid having 16 to 22 carbon atoms.

2. An agent for use in promoting digestion, which agent comprises a monoacylgylcerol having a glycerine of which the hydroxyl group of 1-position, 2-position or 3-position is esterified with an unsaturated fatty acid having 16 to 22 carbon atoms.

3. An agent for use in alleviating heavy stomach, which agent comprises a monoacylgylcerol having a glycerine of which the hydroxyl group of 1-position, 2-position or 3-position is esterified with an unsaturated fatty acid having 16 to 22 carbon atoms.

4. The agent for use according to claim 1 to 3, which agent comprises a monoacylgylcerol having a glycerine of which the hydroxyl group of 1-position is esterified with an unsaturated fatty acid having 16 to 22 carbon atoms.

5. The agent for use according to claim 1 to 4 which is a drug.

6. The agent for use according to claim 1 to 4 which is a food ingredient.

7. The agent for use according to claim 1 to 4 which is a beverage ingredient.

## Patentansprüche

1. Mittel zur Verwendung für die Inhibierung postprandialer GIP-Sekretion, wobei das Mittel ein Monoacylglycerin mit einem Glycerin umfasst, dessen Hydroxylgruppe in der Position 1, der Position 2 oder der Position 3 mit einer ungesättigten Fettsäure mit 16 bis 22 Kohlenstoffatomen verestert ist.

2. Mittel zur Verwendung für die Förderung der Verdauung, wobei das Mittel ein Monoacylglycerin mit einem Glycerin umfasst, dessen Hydroxylgruppe in der Position 1, der Position 2 oder der Position 3 mit einer ungesättigten Fettsäure mit 16 bis 22 Kohlenstoffatomen verestert ist.

3. Mittel zur Verwendung für die Linderung von Völlegefühl, wobei das Mittel ein Monoacylglycerin mit einem Glycerin umfasst, dessen Hydroxylgruppe in der Position 1, der Position 2 oder der Position 3 mit einer ungesättigten Fettsäure mit 16 bis 22 Kohlenstoffatomen verestert ist.

4. Mittel zur Verwendung nach den Ansprüchen 1 bis 3, wobei das Mittel ein Monoacylglycerin mit einem Glycerin umfasst, dessen Hydroxylgruppe in der Position 1 mit einer ungesättigten Fettsäure mit 16 bis 22 Kohlenstoffatomen verestert ist.

5. Mittel zur Verwendung nach den Ansprüchen 1 bis 4, das ein Arzneistoff ist.

6. Mittel zur Verwendung nach den Ansprüchen 1 bis 4, das ein Nahrungsmittelbestandteil ist.

7. Mittel zur Verwendung nach den Ansprüchen 1 bis 4, das ein Getränkbestandteil ist.

## Revendications

1. Agent pour une utilisation dans l'inhibition de la sécrétion postprandiale de GIP, lequel agent comprend un monoacylglycérol ayant une glycérine dont le groupe hydroxyle de la position 1, la position 2 ou la position 3 est estérifié avec un acide gras insaturé comportant 16 à 22 atomes de carbone.

2. Agent pour une utilisation dans l'activation de la digestion, lequel agent comprend un monoacylglycérol ayant une glycérine dont le groupe hydroxyle de la position 1, la position 2 ou la position 3 est estérifié avec un acide gras insaturé comportant 16 à 22 atomes de carbone.

3. Agent pour une utilisation dans le soulagement d'un estomac lourd, lequel agent comprend un monoacylglycérol ayant une glycérine dont le groupe hydroxyle de la position 1, la position 2 ou la position 3 est estérifié avec un acide gras insaturé comportant 16 à 22 atomes de carbone.

4. Agent pour une utilisation selon les revendications 1 à 3, lequel agent comprend un monoacylglycérol ayant une glycérine dont le groupe hydroxyle de la position 1 est estérifié avec un acide gras insaturé comportant 16 à 22 atomes de carbone.

5. Agent pour une utilisation selon les revendications 1 à 4, qui est un médicament.

6. Agent pour une utilisation selon les revendications 1 à 4, qui est un composant alimentaire.

7. Agent pour une utilisation selon les revendications 1 à 4, qui est un composant de boisson.
